(19) Europäisches Patentamt

European Patent Office

Office européen des brevets

(11) **EP 1 690 531 A2**

(12) **EUROPEAN PATENT APPLICATION**

(43) Date of publication:
16.08.2006 Bulletin 2006/33

(51) Int Cl.:
*A61K 9/50* (2006.01)   *A61K 9/20* (2006.01)

(21) Application number: 06006288.2

(22) Date of filing: 16.09.1997

(84) Designated Contracting States:
AT BE CH DE DK ES FI FR GB GR IE IT LI LU MC NL PT SE
Designated Extension States:
AL LT LV RO SI

(30) Priority: 30.09.1996 US 28726 P
12.11.1996 US 30514 P
22.04.1997 US 44121 P

(62) Document number(s) of the earlier application(s) in accordance with Art. 76 EPC:
97910713.3 / 0 932 388

(71) Applicant: ALZA CORPORATION
Palo Alto
California 94303-0802 (US)

(72) Inventors:
• **Gupta, Suneel, K.**
**Sunnyvale, CA 94087 (US)**

• **Christopher, Carol, A.**
**Belmont, CA 94002 (US)**
• **Guinta, Diane, R.**
**Palo Alto, CA 94303 (US)**
• **Saks, Samuel, R.**
**Burlingame, CA 94010 (US)**

(74) Representative: **Golding, Louise Ann**
**Frank B. Dehn & Co.**
**St Bride's House**
**10 Salisbury Square**
**London EC4Y 8JD (GB)**

Remarks:
This application was filed on 27 - 03 - 2006 as a divisional application to the application mentioned under INID code 62.

(54) **Dosage form and method for administering drug**

(57) A dosage form and a method are disclosed and claimed for administering a drug in a sustained and constantly ascending rate per unit time to provide an intended therapeutic effect while concomitantly lessening the development of unwanted effects. In particular, the invention provides a pharmaceutical composition in a dosage form comprising a dose of a central nervous system stimulant, wherein said dosage form is capable of delivering said drug at a sustained and continuously ascending rate.

EP 1 690 531 A2

**Description**

## FIELD OF THE INVENTION

This invention pertains to both a novel dosage form and to a novel method for administering a drug for producing a therapeutic effect. The invention concerns more specifically a dosage form that administers at a sustained and continuously ascending rate a drug to produce a given therapy, and more specifically a method for producing a therapeutic effect by administering over a predetermined period at a sustained and continuously ascending rate a drug to produce a given therapy. The invention relates also to a dosage form and to a method for achieving a therapeutic effect by administering an initial dose of drug followed by a sustained and increasing dose of drug over an extended time.

## BACKGROUND OF THE INVENTION

For a long time, pharmacy and medicine in every society used drugs that produce effects on pain, mood, thought, feeling, behavior, and psychological personality. These drugs are represented by opioids, barbiturates, hypnotics, central nervous system stimulants, central nervous system depressants, psychostimulants, alcohols, cannabinoids, and catecholamines. In present medicine, one class of these drugs has become the standard invention for the management of Attention-Deficit Disorder, that is, the central nervous system stimulants. While this invention presents central nervous system drugs in greater detail, it is understood the invention is generic and embraces drugs broadly administered using the dosage forms and the method of the invention.

The benefits perceived by parents, teachers, physicians, psychologists, social workers, and clinicians are dramatic for central nervous system drugs, and this has resulted in the widespread and acceptable use of central nervous system medication to treat Attention-Deficit Disorder.

In 1994, the latest period for collecting data, it was observed that about two percent of the school-aged female population and about six percent of the school-aged male population, for a total of about two million patients, were administered the medication for Attention-Deficit Disorder.

Prior to the advent of this invention, the dosage form and the method for administering a drug, for example, a central nervous system acting drug, consisted in using a standard pharmaceutical dosage form. For example, one prior art dosage form and method for administering a drug, such as the central nervous system drug methylphenidate, consists in using an immediate release tablet containing the drug. This immediate release form delivers the drug by instant dumping of the drug and this produces uneven blood levels characterized by peaks and valleys. For an immediate release form containing methylphenidate which is characterized by a rapid onset and a short half-life to produce the intended therapy, multiple doses are needed each day that can result in swings in behavior and in attention as the medication loses its therapeutic effect. This dosage form does not provide the needed therapy over an extended time.

Another prior art dosage form for dispensing a drug is the sustained-release dosage form. A drug dispensed from a prior art sustained-release dosage form may ascend initially but not over the entire dosing interval, and it actually may decline over time. That is, these sustained-release dosage forms dispense a drug in a nonascending profile over time, as they do not provide a continuously increasing release rate per hour throughout the extended dosing period. This dosage form, additionally, may not provide the required duration of therapy and the appropriate blood pattern. For drugs that act on the central nervous system, like methylphenidate, dispensed from a sustained-release nonascending dosage form, the patient often develops an acute tolerance to the drug manifested by a shortened duration and a decrease in the intensity of the therapeutic effect needed for acceptable therapy. The prior sustained-release delivery is also devoid of means that compensate for its shortcomings inherent therein.

The above presentation teaches that a critical need exists for a novel dosage form and for a novel method for administering a drug that overcomes the shortcomings known to the prior art This long-felt need exists for a dosage form and for a method for (1) administering the drug at a sustained-increasing rate that simultaneously reduces or eliminates the frequency of daily dosing; for (2) a dosage form and a method for administering the drug in a sustained-compensating dose to substantially compensate for acute tolerance to the drug thereby maintaining a preselected clinical response; for (3) a dosage form that administers the drug in a sustained-ascending profile clinically indicated for the management of Attention-Deficit Disorders; and, for (4) a dosage form and a method for administering the drug initially and in a sustained-ascending profile throughout the entire school day.

## OBJECTS OF THE INVENTION

Accordingly, in view of the above presentation, it is an immediate object of this invention to make available a dosage form that overcomes the shortcomings known in the prior art.

Another object of the invention is to provide a novel and unique dosage form that delivers a drug in a controlled increasing dose to a patient over time.

Another object of the invention is to provide a dosage form that administers a dose of drug to maintain the therapeutic effect of the drug in a patient that acquires tolerance to the drug by delivering the drug in a controlled-increasing dose over time to maintain the therapeutic effect, while concomitantly substantially avoiding the development of acute tolerance.

Another object of the invention is to make available a dosage form that delivers a dose of drug that essentially avoids or lessens the development of acquired tolerance in a patient administered a drug that leads to acute tolerance by the dosage form administering the drug in a sustained and increasing dose over time.

Another object of the invention is to provide a dosage form for administering a central nervous system drug that overcomes the shortcomings known to the prior art.

Another object of the invention is to provide an improvement in a dosage form, wherein the improvement comprises the dosage form administering the drug in a sustained and constantly ascending profile over time for treating Attention-Deficit Disorder.

Another object of the invention is to provide a pharmacological composition as a solid dosage form comprising 1 mg to 500 mg of drug in admixture with a pharmaceutically acceptable carrier that is released in a sustained release and increasing dose for use in the treatment of psychological personalities.

Another object of the invention is to provide a dosage form for increasing the administration of a central nervous system acting drug per hour throughout a school day of 4 to 8-1/2 hours.

Another object of the invention is to provide a dosage form for administering a drug possessing central nervous system therapy in a sustained-increasing rate and at a minimum dose per day.

Another object of the invention is to provide a dosage form for administering a central nervous system acting drug in a drug delivery pattern that compensates for acquired tolerance associated with the drug.

Another object of the invention is to provide a dosage form that administers a drug for treating Attention-Deficit Disorder that comprises administering orally to a human diagnosed as having the disorder the drug in a sustained and increasing dose of 100 ng to 375 mg over 16 hours for treating the disorder in a human.

Another object of this invention is to provide a novel and unique method for maintaining the therapeutic effect of a drug in a patient that acquires tolerance to the drug, wherein the method comprises administering a dosage form to the patient that delivers the drug in a controlled increasing dose over an extended time to maintain the therapeutic effect, while concomitantly substantially avoiding the development of acute tolerance in the patient.

Another object of the invention is to make available a method for essentially avoiding or lessening the development of the acquired tolerance in a patient administered a drug that develops acute tolerance in the patient, wherein the method comprises administering the drug in a sustained and increasing dose over time to produce the intended effect.

Another object of the invention is to provide an improvement in a method for administering a drug, wherein the improvement comprises administering the drug in a sustained and constantly ascending profile over an extended time for treating Attention-Deficit Disorder.

Another object of the invention is to provide a method for administering a central nervous system acting drug in a continuously increasing release rate per hour throughout a school day of 4 to 8-1/2 hours.

Another object of the invention is to provide a novel method to compensate for acute tolerance development associated with a drug possessing the ability to produce tolerance in a patient, by administering the drug orally in a sustained-ascending dose to substantially lessen the unwanted effects of acute tolerance.

Another object of the invention is to provide an improvement in a method for administering a drug possessing central nervous system stimulant therapy, wherein the improvement comprises administering the drug in a sustained and ascending pattern over an extended time for treating Attention-Deficit Disorders and additionally provides therapeutic compensation for acquired tolerance associated with the drug.

Another object of the invention is to provide a method for treating Attention-Deficit Disorder comprising administering orally to a human diagnosed as having the disorder at a sustained and increasing dose of 100 ng to 500 mg over 16 hours a central nervous system drug for treating the disorder in a human patient and at a minimum number of doses per day.

Another object of the invention is to administer a central nervous system acting drug by a method wherein the drug ascends initially and continuously over the entire dosing interval for treating Attention-Deficit Disorder, ADD, and Attention-Deficit Hyperactivity Disorder, ADHD.

These objects, as well as other objects, features, and advantages of the invention will become more apparent from the following detailed disclosure of the invention and the accompanying claims.

## DRAWING FIGURES PROVIDED BY THE INVENTION

In Figure 1, the solid line depicts the plasma concentration for a central nervous system stimulant administered from an immediate release form, and the dotted line denotes the plasma concentration for a central nervous system stimulant released from a sustained nonascending form.

In Figure 2, an immediate release dosage form is depicted by the solid line comprising a peak and a valley depicted against a sustained release ascending dose shown by the dotted line.

In Figures 3, 4, and 5, the release results are depicted for a central nervous system drug wherein the dear circles denote a placebo, the dark circles denote an immediate release form, the dark squares denote a sustained-nonascending release profile, and clear squares denote a sustained ascending release rate profile.

In Figure 6, a sustained-ascending release plasma concentration is depicted by the solid line and compared with an immediate-release plasma concentration depicted by the dash line comprising the peaks and valleys.

In Figure 7, the solid circles denote a placebo, the clear circles a sustained-ascending profile, and the solid squares a three-times-a-day program for the same central nervous system drug.

Figure 8 depicts a placebo given three times a day comprising dark circles, an immediate-release depicted by solid squares, and a sustained-ascending release essentially free of tolerance by clear circles for the same drug.

Figures 9-11 depict the plasma methylphenidate concentration obtained by administering the drug three times a day, in an ascending dose, and by a dosage form that controls the delivery profile.

Figures 12-16 depict the plasma methylphenidate concentration obtained by administering the drug from a dosage form comprising an external overcoat initial dose of drug followed by an internal ascending dose of drug over time.

## DETAILED DISCLOSURE OF SPECIFICATION

In accordance with the practice of this invention, it has now been found that a dosage form and a method can be provided that administers a drug in a novel program that substantially lessens or completely compensates for tolerance in a patient. Tolerance, as defined in Pharmacology in Medicine, by Brill, p. 227 (1965) McGraw-Hill, is characterized as a decrease in effect followed by administering a drug. When tolerance develops following a single dose or a few doses over a very short time, it is referred to as acute tolerance. When the drug is administered over a more protracted period of time to show a demonstrable degree of tolerance, it is referred to as chronic tolerance. The medical literature, as exemplified in, The Pharmacological Bases of Therapeutics, by Goodman and Gilman, 8th Ed., p. 72 (1990) Pergamon Press, reported tolerance may be acquired to the effects of many drugs and this literature classifies tolerance as acute or chronic based on when it is acquired. That is, acute tolerance develops during a dosing phase of one dose or on one day, and chronic tolerance is acquired due to chronic administration typically weeks, months, and years. Tolerance as presented in medical literature most frequently denotes chronic tolerance as seen by administering larger doses over a long time, often necessitated by an increase in body dimensions, hepatic enzyme involved in biotransformation, and the like.

The invention comprises dosage forms for providing an ascending dose of drug. Representative of a dosage form comprises a hydrogel matrix containing a plurality of tiny pills. The hydrogel matrix comprises a hydrophilic polymer selected from the group consisting of a polysaccharide, agar, agarose, natural gum, alkali alginate including sodium alginate, carrageenan, fucoidan, furcellaran, laminaran, hypnea, gum arabic, gum ghatti, gum karaya, gum tragacanth, locust bean gum, pectin, amylopectin, gelatin and a hydrophilic colloid. The hydrogel matrix comprises a plurality of 4 to 50 tiny pills, each tiny pill comprising an increasing dose population of from 100 ng ascending in dose such as 0.5 mg, 1 mg, 1.2 mg, 1.4 mg, 1.6 mg, 1.8 mg, etc. The tiny pills comprise a release rate controlling wall of 0.0 mm to 10 mm thickness to provide for the timed ascending release of drug. Representative of wall-forming materials include a triglyceryl ester selected from the group consisting of glyceryl tristearate, glyceryl monostearate, glyceryl dipalmitate, glyceryl laureate, glyceryl didecenoate and glyceryl tridecenoate. Other wall forming materials comprise polyvinyl acetate phthalate, methylcellulose phthalate, and microporous vinyl olefins. Procedures for manufacturing tiny pills are disclosed in U.S. Patent Nos. 4,434,153; 4,721,613; 4,853,229; 2,996,431; 3,139,383 and 4,752,470.

The dosage form of the invention for delivering an ascending dose of drug comprises drug releasing beads. The drug releasing beads are characterized by a dissolution profile wherein 0 to 20% of the beads undergo dissolution and release the drug in 0 to 2 hours, 20 to 40% undergo dissolution and release the drug in 2 to 4 hours, 40 to 60% exhibit dissolution and release in 4 to 6 hours, 60 to 80% in 6 to 8 hours, and 80 to 100% in 8 to 10 hours. The drug releasing beads comprise a central composition or core comprising a drug and pharmaceutically acceptable composition forming ingredients including a lubricant, antioxidant, and buffer. The beads comprise increasing doses of drug, for example, 1 mg, 2 mg, 5 mg, and 10 mg, increasing to 40 mg. The beads are coated with a release rate controlling polymer that can be selected utilizing the dissolution profile disclosed above. The manufacture of beads is disclosed in Inter. J. of Pharm., by Liu, Vol. 112, pp. 105-116 (1994); Inter. J. of Pharm., by Liu and Yu, Vol. 112, pp. 117-124 (1994); Pharm. Sci., by Remington, 14th Ed. pp. 1626-1628 (1970); J. Pharm. Sci., by Fincher, Vol. 57, pp. 1825-1835 (1968); and U.S. Patent No. 4,083,949.

A dosage form provided by the invention comprises a concentration gradient of drug from 1 mg to 100 mg coated from the former low dose to the latter high dose on a polymer substrate. The polymer can be erodible or a nonerodible polymer. The coated substrate is rolled about itself from the latter high dose at the center of the dosage form, to the former low dose at the exposed outer end of the substrate. The coated substrate is rolled from the high dose to the low dose to provide for the release of from low to high dose as the substrate unrolls or erodes. For example, 1 mg to 25 mg of methylphenidate is coated onto an erodible polymer such as an polypeptide, collagen, gelatin, or polyvinyl alcohol, and the substrate rolled concentrically from the high dose rolled over and inward to adapt a center position, and then outward towards the low dose to form an outer position. In operation, the dosage form erodes dispensing an ascending dose of methylphenidate that is released over time.

Another dosage form provided by the invention comprises a multiplicity of layers, wherein each layer is characterized by an increasing dose of drug. The phrase "multiplicity of layers" denotes 2 to 6 layers in contacting lamination. The multiplicity of layers are positioned consecutively, that is, one layer after another in order, with a first exposed layer, the sixth layer in contact with the fifth layer and its exposed surface coated with a drug impermeable polymer. The sixth layer is coated with a drug impermeable polymer to insure release of drug from the first layer to the sixth layer. The first layer comprises 1 to 5 mg of drug and each successive layer comprises an additional 1 to 5 mg of drug. The biodegradable polymers undergo chemical decomposition to form soluble monomers or soluble polymer units. The biodegradation of polymers usually involves chemically or enzymatically catalyzed hydrolysis. Representative of biodegradable polymers acceptable for an increase drug loading in each layer of from 5 to 50 wt% over the first and successive layers wherein the first layer comprises 100 ng. Representative biodegradable polymers comprise a member selected from the group consisting of biodegradable poly(amides), poly(amino acids), poly(esters), poly(lactic acid), poly(glycolic acid), poly (orthoesters), poly(anhydrides), biodegradable poly(dehydropyrans), and poly(dioxinones). The polymers are known to the art in Controlled Release of Drugs, by Rosoff, Ch. 2, pp. 53-95 (1989); and in U.S. Patent Nos. 3,811,444; 3,962,414; 4,066,747; 4,070,347; 4,079,038; and 4,093,709.

The invention further employs a dosage form comprising a polymer that releases a drug by diffusion, flux through pores, or by rupture of a polymer matrix. The drug delivery polymeric system comprises a concentration gradient, wherein the gradient is an ascent in concentration from a beginning or initial concentration to a final, or higher concentration of 100 ng to 250 mg. The dosage form comprises an exposed surface at the beginning dose and a distant nonexposed surface at the final dose. The nonexposed surface is coated with a pharmaceutically acceptable material impermeable to the passage of drug. The dosage form structure provides for a flux increase delivery of drug ascending from the beginning to the final delivered dose.

The dosage form matrix can be made by procedures known to the polymer art. In one manufacture, 3 to 5 or more casting compositions are independently prepared wherein each casting composition comprises an increasing dose of drug with each composition overlayered from a low to the high dose. This provides a series of layers that come together to provide a unit polymer matrix with a concentration gradient. In another manufacture, the higher does is cast first followed by laminating with layers of decreasing dose to provide a polymer matrix with a drug concentration gradient. An example of providing a dosage form comprises blending a pharmaceutically acceptable carrier, like polyethylene glycol, with a known dose of drug, like a central nervous system stimulant, at an elevated temperature, like 37°C, and adding it to a silastic medical grade elastomer with a cross-linking agent, like stannous octanoate, followed by casting in a mold. The step is repeated for each successive layer. The system is allowed to set, for 1 hour, to provide the dosage form. Representative polymers for manufacturing the dosage form comprise a member selected from the group consisting of olefin and vinyl polymers, condensation polymers, carbohydrate polymers, and silicon polymers as represented by poly(ethylene), poly(propylene), poly(vinyl acetate), poly(methyl acrylate), poly(isobutyl methacrylate), poly(alginate), poly(amide), and poly(silicone). The polymers and manufacturing procedures are known in Polymers, by Coleman et al., Vol. 31, pp. 1187-1230 (1990); Drug Carrier Systems, by Roerdink et al., Vol. 9, pp. 57-109 (1989); Adv. Drug Delivery Rev., by Leong et al., Vol. 1, pp. 199-233 (1987); Handbook of Common Polymers, Compiled by Roff et al., (1971) published by CRC Press; and U.S. Patent No. 3,992,518.

Further in accord with the practice of the present invention, the method of this invention uses the disclosed dosage forms for administering a drug to a patient that may acquire acute or chronic tolerance for decreasing and/or avoiding said tolerance, and presently the method is indicated for treating patients that may acquire acute tolerance. Further, in accordance with the practice of this invention, in one embodiment, it has also been found a method can be provided that administers a drug for treating Attention-Deficit Disorder, to a human orally as a function of time to achieve the desired drug concentration over time. The concentration of drug relates to the dose of drug in mg per hour delivered per unit time in hours for absorption into the systemic circulation. The method of the invention uniquely provides a method for maintaining a desired drug effect by adjusting continually the drug delivery rate when the therapeutic effect declines during acquired acute tolerance.

It is standard medical practice, that a drug should provide a therapeutic effect throughout the dosing interval. However, when tolerance develops or is acquired to a drug, the prior art approach to ensure a therapeutic response is to increase the dose administered in an immediate dose dumping manner, and for this type of dosing, where associated side effects are likely to occur, tolerance may develop unequally to all the effects, and the therapeutic index may decrease. Another approach used by the prior art to lessen the occurrence of tolerance is to administer drug doses less frequently so that acquired tolerance is avoided, but with this approach there is an absence of therapy for a given time.

In medicine, it is generally accepted that central nervous system acting drugs are useful for the management of Attention-Deficit Disorders. The drugs useful for this therapy are the mild central nervous system stimulants, and they include catecholamines and drugs that can mimic their action. The drugs useful for this therapy comprise a member selected from the group consisting of amphetamine, dextro-amphetamine, methamphetamine, methylphenidate, racemic methylphenidate, threo-methylphenidate, phenylisopropylamine, risperidone, and pemoline. The drugs include also their pharmaceutically acceptable salts such as a member selected from the group consisting of hydrochloride, sulfate, phosphate, acetate, hydrobromide, pamoate, and maleate. A patient receiving these drugs typically acquires tolerance to the effects of the drugs. For example, a patient on methylphenidate and receiving a 5 mg dose twice a day acquires tolerance, and a larger dose must be administered due to the single large dose needed to overcome the tolerance development which would give rise to unwanted side effects. In some patients, the therapeutic response to methylphenidate declines in 4 to 5 hours despite the maintenance of methylphenidate in a nonascending constant concentration. That is, tolerance is acquired to the behavioral and psychological effects of methylphenidate and generally to psychostimulants. This invention has found also that a sustained release product that dispenses a noncompensating but constant concentration of a drug will not be clinically effective, as a sustained-release dosage form designed to produce a constant plasma of, for example, methylphenidate concentration, lacks efficacy particularly against acquired tolerance.

This invention among its objects provides a dosage form and method for treating Attention-Deficit Disorders, which include Attention-Deficit/Hyperactivity Disorder, combined type, predominantly inattentive type, predominantly hyperactive impulsive type and which are known also as minimal brain dysfunction, hyperkinetic child syndrome, behavioral syndrome, minimal cerebral dysfunction and minor cerebral dysfunction, as disclosed in the Diagnostic and Statistical Manual of Mental Disorders, 3rd Edition, pp. 49-56 (1987) published by the American Psychiatric Association, Washington, D.C., by making available both a dosage form and a method of treatment that substantially negates the unwanted results of the prior art by providing continuous compensation of drug to essentially eliminate acute tolerance, thereby producing a stabilization of the therapeutic effect.

The pharmacological effect of a drug is related to its receptor site concentration. Thus, when the effect of a drug is considered as a function (f) of delivery time (t), the kinetics of stabilization for some drugs can be rapid, and for other drugs the effect does not stabilize as a diminution in response expressed as tolerance develops to the drug. This latter condition applies to central nervous system acting drugs such as methylphenidate. This invention compensates for acquired-acute tolerance by providing an optimal drug delivery profile for its management. For some drugs the onset of tolerance is quick, for instance, tolerance develops for methylphenidate within hours after its administration. A management program for this is provided by this invention by making available a drug delivery pattern that initially delivers a dose of drug to achieve instant therapy and accompanied by a sustained-ascending release dose over time to maintain the effect.

The drug methylphenidate is commercially available in a sustained release dosage form Ritalin® -SR, and the methylphenidate dispensed by this commercial form can lead to acute tolerance. When acute tolerance is acquired a drug-free washout period of several hours is needed before a repeat administration is likely with the dosage form. The present invention however, compensates for the loss of a therapeutic effect of a drug, such as methylphenidate, by providing a method of delivery rate in mg per hour that continually compensates for the development of acute tolerance, by considering the clinical effect (E) of a drug at time (t) as a function of the drug concentration (C) according to Equation 1:

$$\text{Effect } = f(t, C) \qquad\qquad \text{Eq. 1}$$

In addition, the rate of drug delivered (A), in mg per hour is directly proportional to the concentration times the clearance of the drug. As the effect varies with time and the functionality is expressed, then according to this invention (A) can be governed to ensure the therapeutic effect is maintained at a clinical value. If the effect from a drug is found clinically to decrease with time, this decline could be linear as expressed by Equation 2:

$$\text{Effect}_{(t)} = \text{Effect}_{(ini)} - k_{Effect} * t \qquad\qquad \text{Eq. 2}$$

wherein, Effect $_{(ini)}$ is the clinical effect observed initially at the start of drug administration and Effect $_{(t)}$ is the effect observed at time (t) hours, $k_{effect}$ is a proportionality constant ascertained by measuring the clinical effect (E1) at time (t1) hours and (E2) at time (t2) hours while maintaining a constant plasma concentration followed by dividing (E1) minus (E2) by (t1) minus (t2). In order to maintain a constant effect, (A) must be adjusted with the same functionality according to Equation 3:

$$A_{(t)} = A_{(ini)} + k_{Effect} * t \qquad \text{Eq. 3}$$

wherein $A_{(ini)}$ is the initial drug input in mg per hour at the start of the therapy and $A_{(t)}$ is the drug input at time (t) hours, and $k_{Effect}$ is the proportionality constant presented above. If the therapeutic effect is found to decline exponentially with time, this relationship is expressed by Equation 4:

$$Effect_{(t)} = Effect_{(ini)} * exp^{(-kEffect*t)} \qquad \text{Eq. 4}$$

wherein $Effect_{(ini)}$ and $Effect_{(t)}$ are as defined before, $k_{Effect}$ is a rate constant ($h^{-1}$), a unit of reciprocal hours, ascertained by measuring the clinical effect (E1) at time (t1) hours and (E2) at time (t2) hours while maintaining a constant plasma concentration followed by dividing natural log of (E1) minus natural log of (E2) by (t1) minus (t2). To maintain a constant effect, (A) must be adjusted according to Equation 5:

$$A_{(t)} \simeq A_{(ini)} * exp^{(kEffect*t)} \qquad \text{Eq. 5}$$

wherein $A_{(ini)}$ and $A_{(t)}$ is as defined before. $k_{Effect}$ is the rate constant ($h^{-1}$) presented above. The equations are presented in Pharmac. Ther., Vol. 16, pp. 143-166 (1982) by Holford N.H.G. and Sheiner, L.E.

The effects defined herein refer to the pharmacological effects exhibited by the drug as ascertained by clinical subjective observation such as SKAMP and CLAM, or as ascertained by objective activity monitoring such as mathematic tests and school accomplishments. The CLAM Test is a behavior rating that indicates social conformity or rebellion as developed by Conners, Lonez, and Milch and SKAMP is a rating that also measures behavior as developed by Swanson and reported in Psychopharmacological Bulletin, Vol. 21, pp. 887-890 (1985).

The effect measurements in this study were: (1) observer ratings on SKAMP scale (during classroom time) and (2) performance on the computerized mathematics test. Each child was tested on the mathematics test before the study began, and based on this pre-test during the study, a mathematics test appropriate for each child's ability was given. The morning and evening parent CLAM assessments were used to identify unusual behaviors. The evening parent CLAM was used to determine the presence of treatment effects in the evening hours, particularly treatment effects on the time the child fell asleep, and whether the child's sleep was interrupted. All of the children also wore an activity monitor (Actigraph) which records the movements of the child throughout the day. The activity (number of movements per minute) is recorded electronically and modeled as a function of the drug effect.

## DETAILED DISCLOSURE OF EXAMPLES

## PROVIDED BY THE INVENTION

The following examples are merely illustrative of the present invention and they should not be considered as limiting the scope of this invention in any way, as these examples and other equivalents thereof will become apparent to those versed in the art in the light of the present disclosure and the accompanying claims.

## EXAMPLE 1

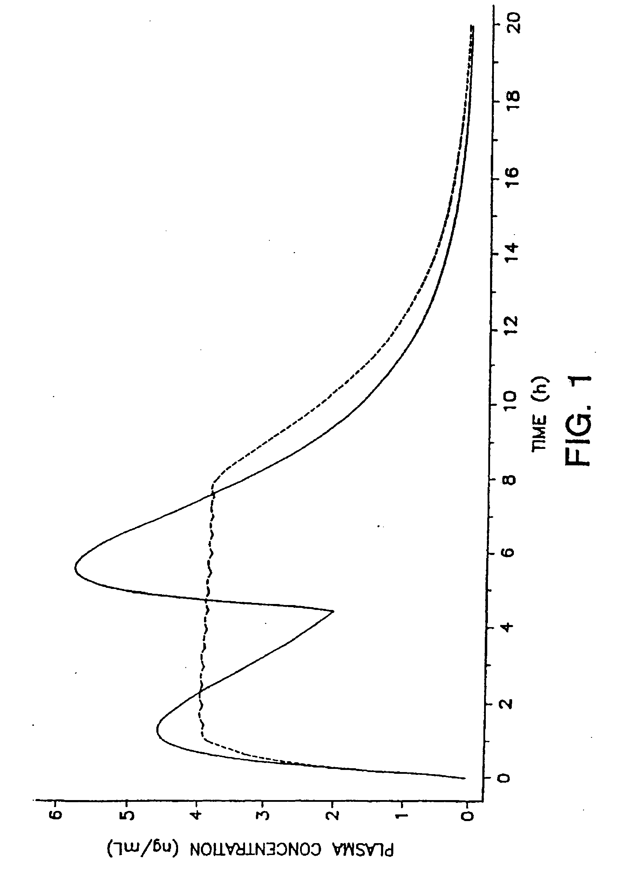

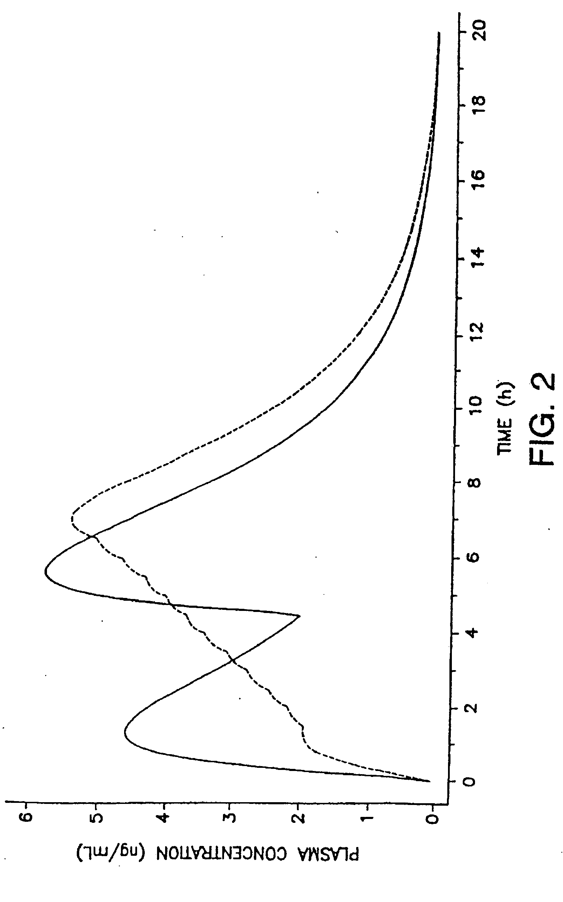

A commercially available immediate release tablet consisting of 5 mg of methylphenidate was administered twice a day to 36 school children, and the predicted plasma concentration in ng/ml graphed against time as seen in the solid black line in Figure 1. The tablet exhibits a peak and valley plasma concentration for the methylphenidate. A sustained-release nonascending program that administered 20 mg of methylphenidate consisting of 8.3 mg at zero hour followed by 0.9 mg at 1.5 hours, 0.9 mg at 2 hours, 0.9 mg 2.5 hours, 0.9 mg at 3 hours, 0.9 mg at 3.5 hours, 0.9 mg at 4 hours, 0.9 mg at 4.5 hours, 0.9 mg at 5 hours, 0.9 mg at 5.5 hours, 0.9 mg at 6 hours, 0.9 mg at 6.5 hours, 0.9 mg at 7 hours, and 0.9 mg at 7.5 hours produced the sustained-release dotted line parallel to the x-axis as seen in Figure 1. The immediate release tablet and the sustained-release dosage form were compared to a sustained-release dosage form that administered methylphenidate in an ascending profile. The sustained-release ascending profile corresponds to administering 4.2 mg at zero hour, 1.1 mg at 1.5 hours, 1.1 mg at 2 hours, 1.2 mg at 2.5 hours, 1.2 mg at 3.0 hours, 1.3 mg at 3.5 hours, 1.3 mg at 4 hours, 1.5 mg at 4.5 hours, 1.5 mg at 5 hours, 1.8 mg at 5.5 hours, 1.8 mg at 6 hours, and 2.0 mg at 6.5 hours, to produce the sustained-ascending release dotted line profile seen in Figure 2.

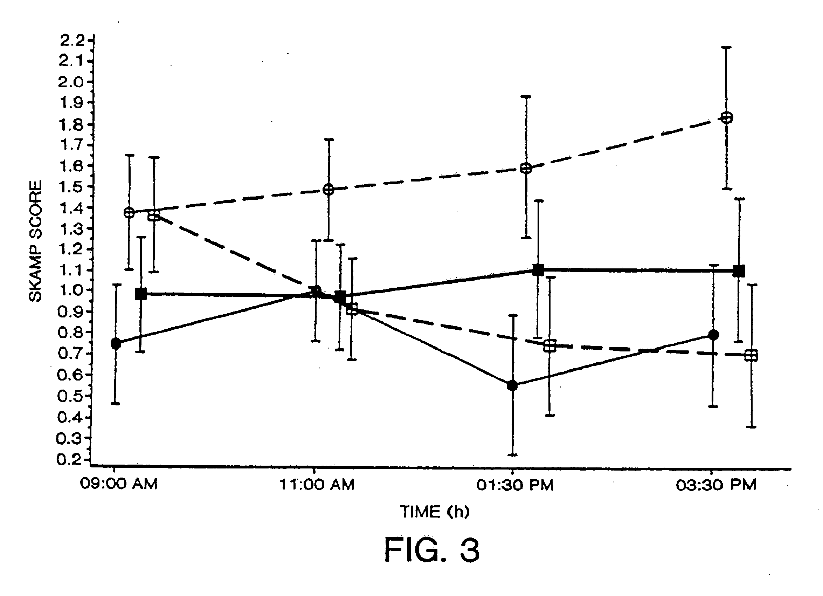

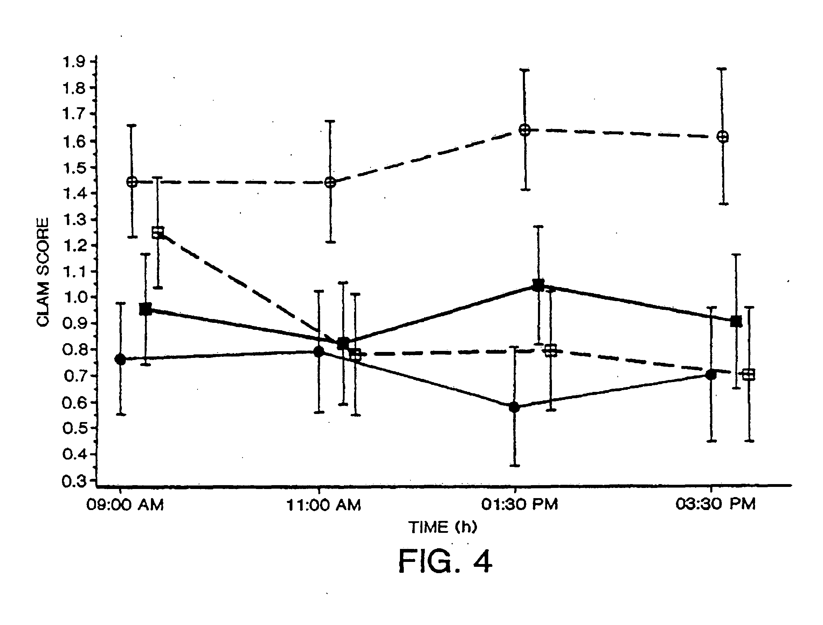

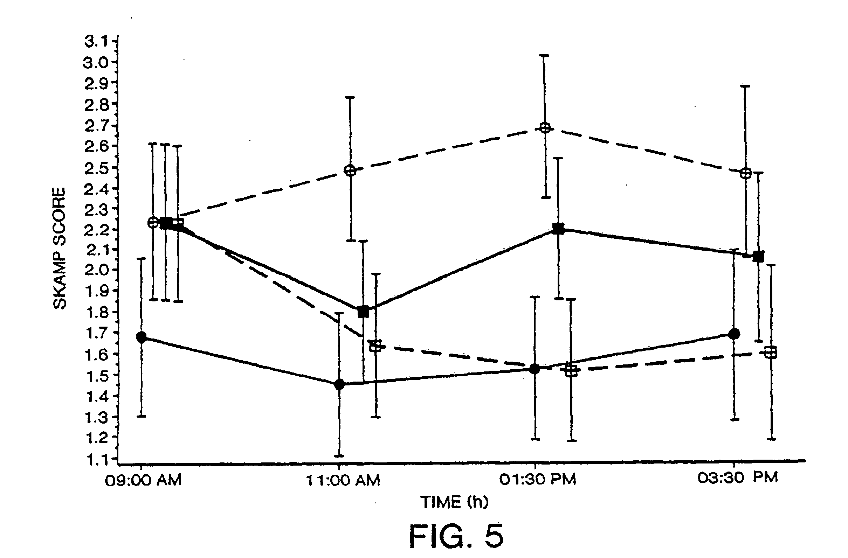

The results of the clinical studies demonstrated patients administered a dosage form free of methylphenidate, a placebo, exhibited high, elevated swings in behavior, such as activity, inappropriate behavior, low attention, lower mathematical scores and a disinterest in school. The patients administered a sustained-nonascending dose of methylphenidate exhibited a decrease in activity, higher mathematical scores and a lessening of inappropriate behavior. However, these effects were accompanied by the development of acute tolerance in the patient. The patient administered methylphenidate, according to this invention, in a controlled-sustained ascending profile exhibited the desired therapeutic effect without tolerance. The accompanying figures present the results of the above-described study. In Figures 3, 4, and 5, the line with a clear circle denotes a placebo, the dark circle an immediate release dosage form administered twice a day, the dark squares a sustained release nonascending dosage profile, and the clear squares the sustained ascending release profile provided by this invention. The SKAMP Score and CLAM Score were defined earlier in the specification, and the times are as indicated on the figures. Figure 3 denotes the observed behavior, Figure 4 denotes the inattention overactivity, and Figure 5 denotes the combined attention results of the study.

## EXAMPLE 2

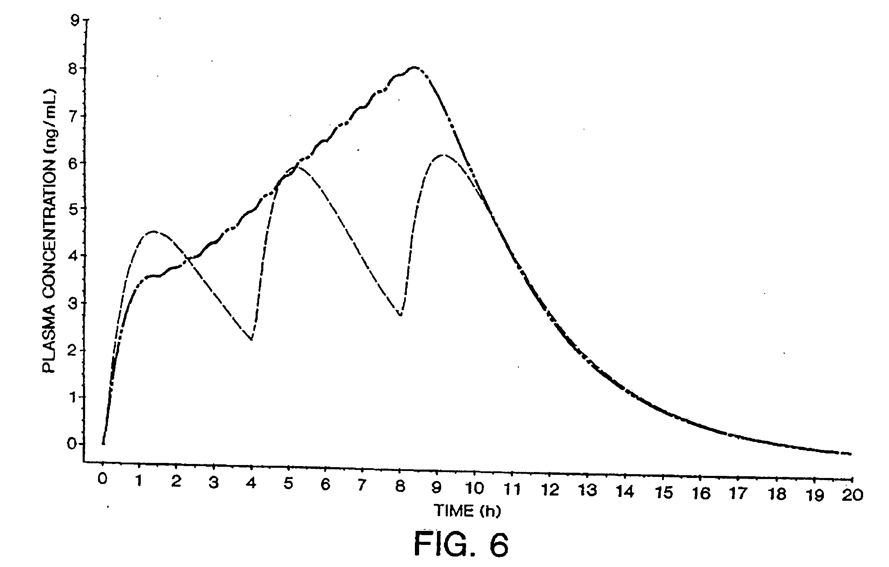

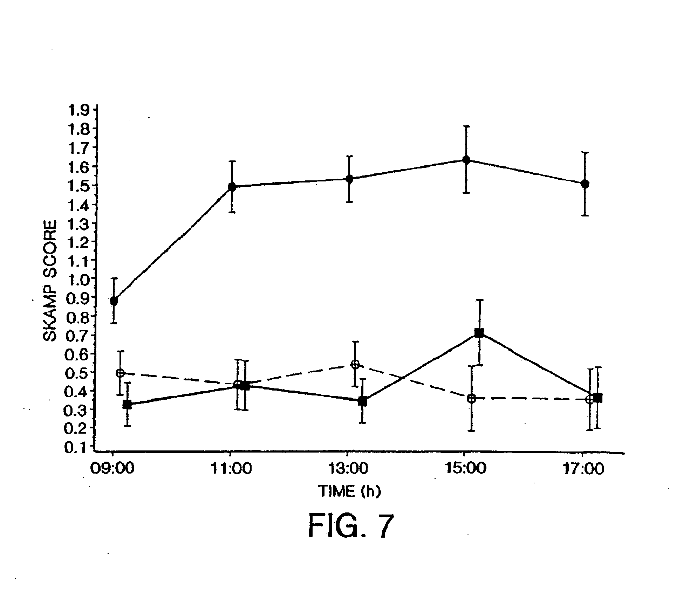

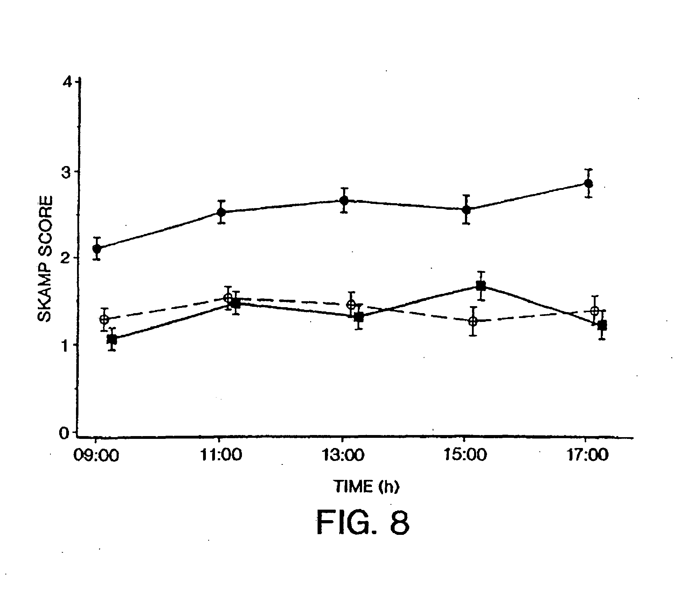

The results of a clinical study that comprises administering methylphenidate according to two distinct delivery programs are reported in this example. In the study, a sustained-ascending profile corresponds to administering methylphenidate as follows: 8 mg at zero hours, 1.4 mg at 1.5 hours, 1.4 mg at 2.0 hours, 1.7 mg at 2.5 hours, 1.7 mg at 3.0 hours, 2.0 mg at 3.5 hours, 2.0 mg at 4.0 hours, 2.2 mg at 4.5 hours, 2.2 mg at 5.0 hours, 2.2 mg at 5.5 hours, 2.2 mg at 6.0 hours, 2.4 mg at 6.5 hours, 2.4 mg at 7.0 hours, 2.6 mg at 7.5 hours, and 1.9 mg at 8.0 hours. The methylphenidate was administered in overcoated capsules comprising a total of 36 mg of methylphenidate with 22% in the exterior overcoat. The ascending dose was administered with the first dose at 0730 followed by ascending doses every 30 minutes until 1530 to produce the intended ascending plasma concentration. The study included the delivery of methylphenidate in immediate dosage form three times a day with 10 mg of methylphenidate delivered at 0730, 1130, and 1530 hours. The study was done with 32 children with attention deficient hyperactivity disorder. Accompanying Figure 6 depicts the plasma concentration for methylphenidate wherein the dot-dash line is produced by the sustained-ascending administration program, and the dash line is produced by the immediate dosage form. In accompanying Figure 7, the solid circles denote placebos, the clear circles denote the sustained-ascending program, the solid squares denote the three times a day program, and the parameter observed was behavior with an absence of acquired tolerance for the sustained ascending program. Accompanying Figure 8 depicts the combined attention parameter wherein the solid circle is a placebo with acquired tolerance, the solid square is the three daily immediate release delivery with acquired tolerance, and the clear circle is the sustained-ascending release essentially free of developed tolerance.

## EXAMPLE 3

A method for administering the central nervous system stimulant methylphenidate in a sustained and ascending dose for the management of attention deficient disorder accompanied by a lessening of acquired tolerance is provided by administering a dosage form shaped as an orally administered tablet. The dosage form comprises a film of polyanhydride polymer of sebacic and azelaic acids coated with a composition comprising 20 mg of methylphenidate blended with pharmaceutically acceptable gelatin. The pharmaceutically acceptable film is coated with the methylphenidate composition in increased thickness spirally wound about itself. Following oral administration into the gastrointestinal tract the composition comprising the methylphenidate is dispensed at constantly increasing rate as the film erodes over time. The polymer of the dosage form is described in U.S. Patent Nos. 2,668,162 and 2,676,945, and the dosage form is described in U.S. Patent No. 3,625,214.

## EXAMPLE 4

A method for treating Attention-Deficit Disorder with hyperactivity supported by psychological and educational guidance by administering pemoline for a stabilizing effect in children accompanied by an apparent absence of acquired tolerance is provided by administering a bioerodible dosage form according to the invention, comprising the central nervous stimulant pemoline. The dosage form comprises 5 contacting layers of bioerodible poly(lactide-co-glycolide) with each layer containing an increased amount of 4, 6, 8, 10 and 12 mg of pemoline. The layers are compressed into a laminated tablet-shaped arrangement with a single opened surface to expose the layer containing 2 mg of pemoline with the remainder of the tablet surrounded with nonbioerodible copolymeric ethylene-vinyl acetate. So the layers bioerode in constant succession, a corresponding constantly increasing dose of pemoline is dispensed over time. The bioerodible polymers are known in U.S. Patent No. 3,773,919; EPO 0-052-510; and Canadian Patent No. 1,169,090.

## EXAMPLE 5

A method for administering a drug in a sustained-increasing release rate is provided by administering a dosage form manufactured as a pharmaceutically acceptable gelatin two-piece joined capsule comprising a multiplicity of spherical beads. The capsule comprises a series of beads consisting of a progression of 1, 1.25, 1.5, 1.75, 2 and 2.25 mg of drug in each different bead coated correspondingly with a progression of 0.5, 1, 1.5, 2.5, 3, and 3.25 mm of polymeric poly(2.2-dioxo-trans-1, 4-cyclchexane dimethylene tetrahydrofuran). As the beads erode in the environment of the gastrointestinal tract they dispense drug in a sustained-ascending release rate over time. The drugs that can be dispensed by this method comprise a member selected from the group consisting of amphetamine, dextroamphetamine, methamphetamine, methylphenidate, phenylisopropylamine and pemoline. Procedures for coating are disclosed in J. Am. Phar. Assoc., Sci. Ed., Vol. 48, pp. 451-454 (1959); and U.S. Patent No. 2,799,241.

## EXAMPLE 6

A delivery system is provided by the invention which releases the drug resulting in an ascending plasma methylphenidate concentration time profile that substantially overcomes tolerance and maintains the desired pharmacological effect of the stimulant methylphenidate for the desired duration. For example, to achieve an effect-time profile similar to the three doses of immediate release given every 4 hours for 12 hours every day, TID (three times a day), a delivery system which results in the plasma methylphenidate concentrations between the ranges as listed below will overcome tolerance and maintain pharmacological effects. To make a delivery system which is equal to two doses of immediate release given every 4 hours the release rate can be truncated, and similarly, for the longer duration the concentration can be increased. The delivery profile exemplifies the drug and pharmacological effect. However, the concept of increasing concentration still remains the same.

Table 1 provides this range as a fraction of the simulated TID concentrations. The attached figures show the ascending profile variations superimposed on the TID and the reference ascending (ASCEND) treatment profiles.

| Time (h) | TID Concentration (ng/Ml) | Ascending Profile Range (Fraction of TID Concentration) | |
|---|---|---|---|
| | | Low | High |
| 1.5 | 4.8 (peak) | 0.75 | 0.90 |
| 3.0 | 3.8 | 1.07 | 1.37 |
| 4.0 | 2.8(trough) | 1.32 | 2.29 |
| 5.5 | 6.5(peak) | 0.80 | 1.20 |
| 7.0 | 4.8 | 1.42 | 1.81 |
| 8.0 | 3.6(trough) | 2.17 | 2.50 |
| 9.5 | 7.0(peak) | 1.10 | 1.23 |
| 11.0 | 5.2 | 1.00 | 1.38 |
| 12.0 | 3.9 | 0.97 | 1.54 |
| 15.0 | 1.7 | 1.00 | 1.94 |

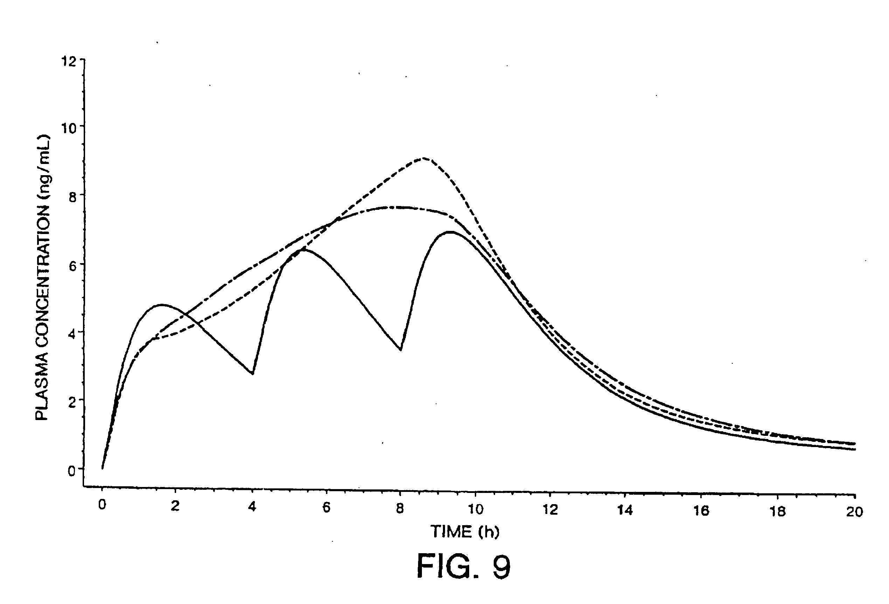

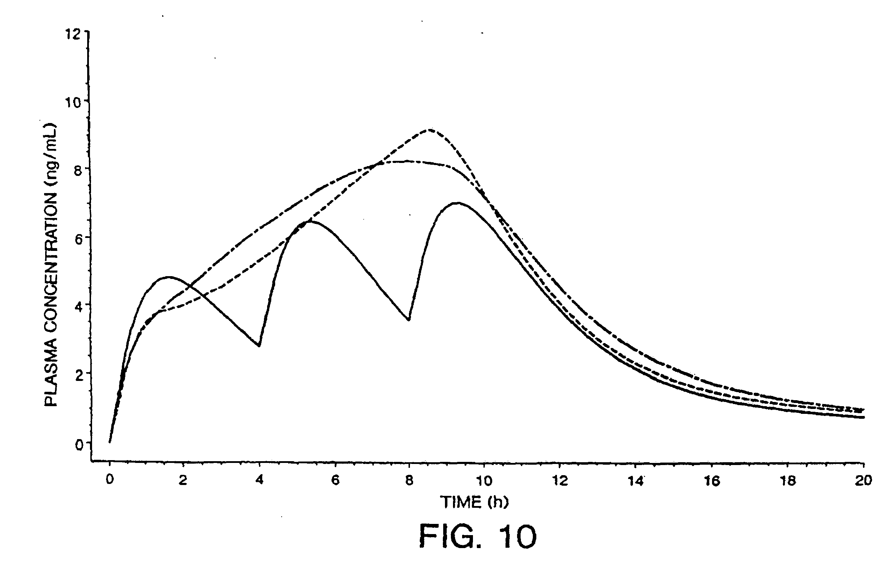

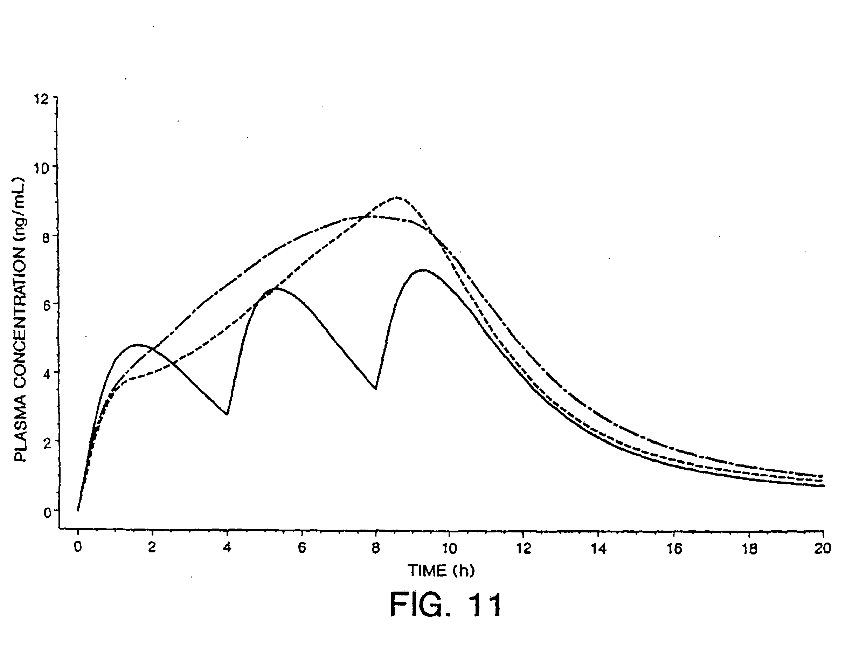

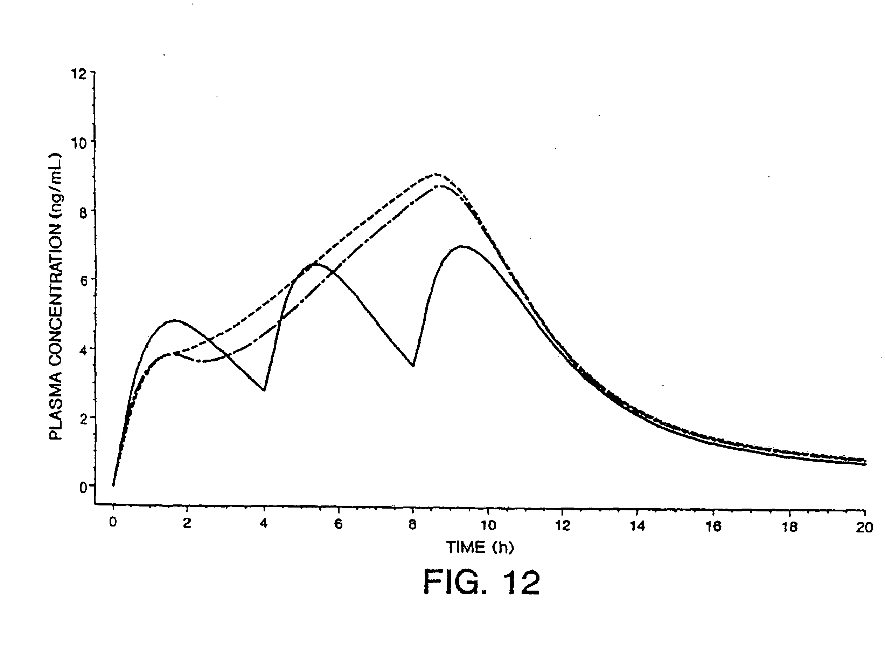

The accompanying drawing figures depict the therapeutic benefits obtained by the invention. Figure 9 illustrates a simulated plasma methylphenidate concentration profile for three-times-a-day 30 mg dose (solid line), an ascend treatment of 36 mg (dash line), and an osmotic controlled 36 mg dose (dot-dash line). Figure 10 depicts the plasma methylphenidate concentration as in Figure 9, except in Figure 10 the osmotic controlled dose is 38 mg. Figure 11 depicts the plasma methylphenidate concentration as in Figure 9, except in Figure 11, the osmotic controlled dose is 40 mg. Figure 12 illustrates 30 mg delivered three times a day by the solid line, an ascend dose from a dosage form comprising 36 mg of drug once a day by the dash line, and a dosage form comprising an immediate 8 mg dose and a sustained 26 mg ascending dose illustrated by the dot-dash line.

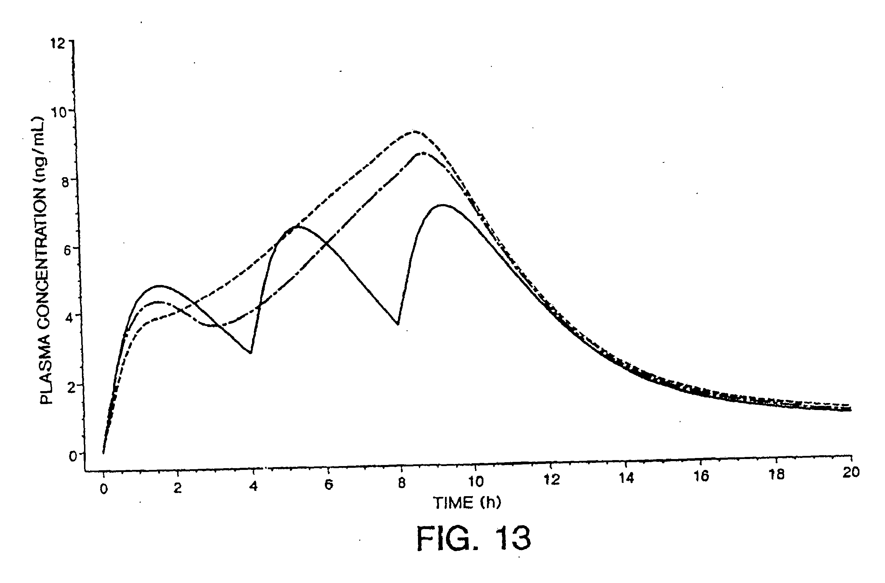

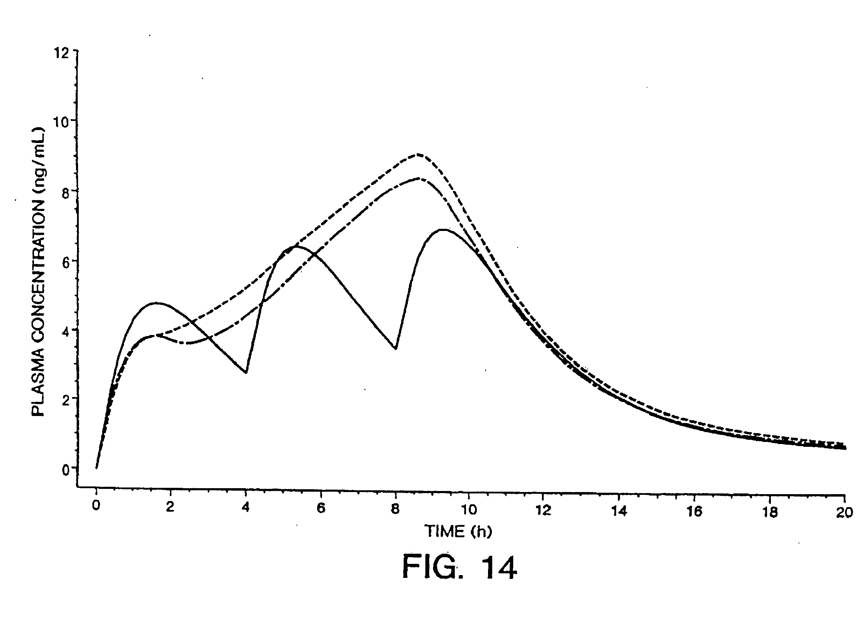

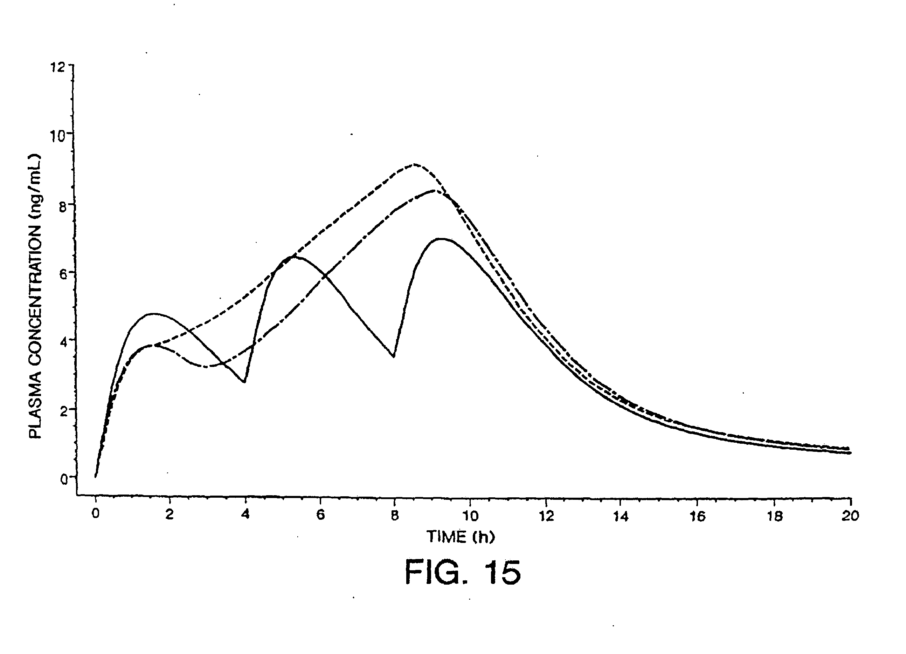

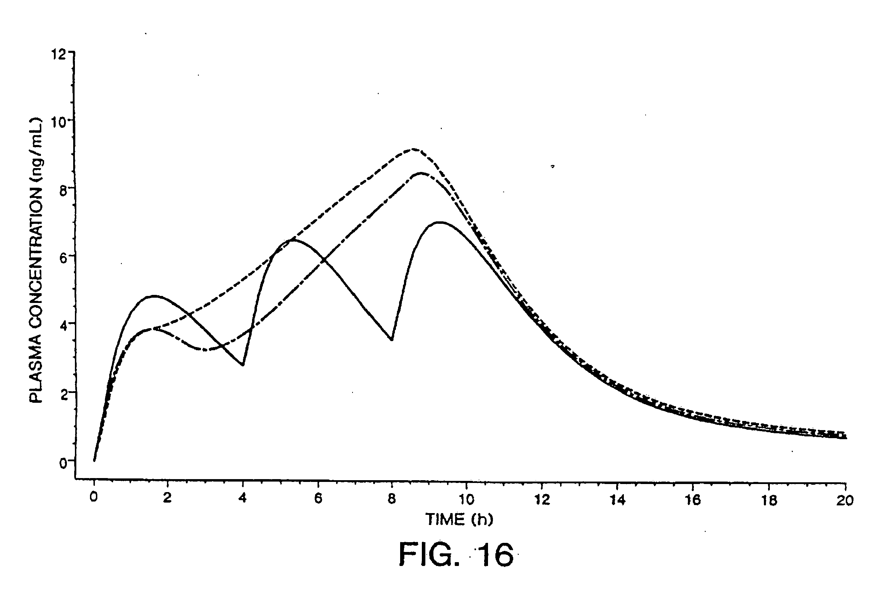

Figure 13 depicts a plasma methylphenidate concentration like Figure 12, except the dosage form represented by the dot-dash line comprises an instant-release dose of 9 mg of methylphenidate and an ascending dose of 24 mg of methylphenidate. Figure 14 is similar to the previous Figures except the dot-dash line depicts an instant release dose of 8 mg and an ascending dose of 25 mg of methylphenidate. Figure 15 is similar to the above Figures, except the dot dash line illustrates an immediate dose of methylphenidate of 8 mg followed by a sustained ascending dose of 25 mg of methylphenidate. Figure 16 is similar to the above Figures with the clinical conditions as set forth, except in this study the dot-dash lines illustrate an immediate dose of 8 mg of methylphenidate, followed by a controlled-ascending dose of 24 mg of methylphenidate.

The method of the invention provides further for administering a drug according to the above examples, wherein the drug is administered by the dosage form of this invention in a controlled-rate and in a sustained release pattern throughout a school day of up to 8 hours, or up to 12 hours.

While there has been described and pointed out features and advantages of the invention, as applied to present embodiments, those skilled in the medical art will appreciate that various modifications, changes, additions, and omissions in the method described in the specification can be made without departing from the spirit of the invention.

Preferred embodiments of the invention include the following:

1. A pharmaceutical composition in a dosage form, comprising a dose of drug in a concentration gradient from a lower to a higher dose that is released in a lower to a higher dose by the dosage form.

2. The dosage form for delivering a drug according to claim 1, wherein the dosage form comprises: a composition comprising a polymer, a dose of drug in the composition present in a concentration gradient from a low to a higher dose, and wherein the dosage form when in operation releases a low followed by a higher dose of drug.

3. The dosage form for delivering a drug according to claim 1, wherein the dosage form comprises: a multiplicity of layers of a composition comprising a polymer, a dose of drug in an increasing dose in the multiplicity of layers, and wherein when the dosage form is in operation, the dosage form delivers an increasing dose of drug over time.

4. The dosage form for delivering a drug according to claim 1, wherein the dosage form comprises: a plurality of layers comprising a composition comprising a different polymer, a dose of drug in an increasing dose in the plurality of layers, and wherein when the dosage form is in operation, the dosage form delivers an increasing dose of drug over time.

5. The dosage form for delivering a drug according to claim 1, wherein the dosage form comprises: a composition comprising a bioerodible polymer, a dose of drug in the composition present in an initial dose and a final dose, whereby the dosage form delivers an initial dose and a final dose over time.

6. The dosage form for delivering an ascending dose of drug according to claim 1, wherein the dosage form comprises: a multiplicity of layers comprising a bioerodible polymer, a drug in an ascending dose in the layers, whereby the dosage form delivers an ascending dose of drug over time.

7. The dosage form for delivering an ascending dose of drug according to claim 1, wherein the dosage form comprises a plurality of layers comprising a different bioerodible polymer, a drug in an ascending dose in the different layers, whereby the dosage from delivers an ascending dose of drug over time.

8. The dosage form for delivering an ascending dose of drug according to claim 1, wherein the dosage form comprises tiny pills comprising a drug, which tiny pills release an initial dose of drug and successive ascending doses of drug over time.

9. The pharmaceutical composition as a dosage from according to claim 1, wherein the pharmaceutical composition comprises a dose of drug in admixture with a pharmaceutically acceptable carrier that is released in a sustained release and increasing dose.

10. The pharmaceutical composition according to claim 1, wherein the pharmaceutical composition comprises 1 mg to 500 mg of drug.

11. The pharmaceutical composition according to claim 1, wherein the drug is released in a sustained and increasing dose of 100 ng to 375 mg over 16 hours.

12. The use of the pharmaceutical composition of claim 1, for the treatment of psychological disorders.

**Claims**

1. A pharmaceutical composition in a dosage form comprising a dose of a central nervous system stimulant, wherein said dosage form is capable of delivering said drug at a sustained and continuously ascending rate.

2. A composition as claimed in claim 1, wherein said dosage form is capable of delivering said drug at a sustained and continuously ascending rate over a period of up to 12 hours.

3. A composition as claimed in claim 1, wherein said dosage form is capable of delivering said drug at a sustained and continuously ascending rate over a period of up to 8 hours.

4. A composition as claimed in claim 1, wherein said dosage form is capable of delivering said drug at a sustained and continuously ascending rate over a period of 4 to 8½ hours.

5. A composition as claimed in any one of claims 1 to 4, wherein said central nervous system stimulant is amphetamine, dextro-amphetamine, methamphetamine, methylphenidate, racemic methylphenidate, threo-methylphenidate, phenylisopropylamine, respiridone, pemoline or a pharmaceutically acceptable salt thereof.

6. A composition as claimed in any one of claims I to 4, wherein said central nervous system stimulant is methylphenidate or a pharmaceutically acceptable salt thereof.

7. A composition as claimed in claim 5 or claim 6, wherein said pharmaceutically acceptable salt is selected from hydrochloride, sulfate, phosphate, acetate, hydrobromide, pamoate and maleate.

8. A composition as claimed in any one of claims 1-6, wherein said dosage form is capable of delivering said drug in an initial dose followed by a sustained and increasing dose over time.

9. A composition as claimed in any preceding claim, wherein said dosage form comprises a hydrogel matrix containing a plurality of tiny pills.

10. A composition as claimed in claim 9, wherein the hydrogel matrix comprises a hydrophilic polymer selected from the group consisting of a polysaccharide, agar, agarose, natural gum, alkali alginate, carrageenan, fucoidan, furcellaran, laminaran, hypnea, gum arabic, gum ghatti, gum karaya, gum tragacanth, locust bean gum, pectin, amylopectin, gelatin and a hydrophilic colloid.

11. A composition as claimed in claim 9 or claim 10, wherein the hydrogel matrix comprises 4 to 50 tiny pills, each tiny pill comprising an increasing dose population of from 100 ng of said drug.

12. A composition as claimed in claim 11, wherein the increasing dose population comprises doses selected from 0.5 mg, 1 mg, 1.2 mg, 1.4 mg, 1.6 mg and 1.8 mg of said drug.

13. A composition as claimed in any one of claims 9 to 12, wherein the tiny pills comprise a release rate controlling wall up to 10 mm thickness.

14. A composition as claimed in claim 13, wherein the release rate controlling wall comprises a triglyceryl ester selected from the group consisting of glyceryl tristearate, glyceryl monostearate, glyceryl dipalmitate, glyceryl laureate, glyceryl didecenoate and glyceryl tridecenoate.

15. A composition as claimed in claim 13, wherein the release rate controlling wall comprises a material selected from polyvinyl acetate phthalate, methylcellulose phthalate and microporous vinyl olefins.

16. A composition as claimed in any one of claims 1 to 8, wherein said dosage form comprises drug releasing beads.

17. A composition as claimed in claim 16, wherein said beads are **characterized by** a dissolution profile wherein 0 to 20% of the beads undergo dissolution and release the drug in 0 to 2 hours, 20 to 40% undergo dissolution and release the drug in 2 to 4 hours, 40 to 60% exhibit dissolution and release in 4 to 6 hours, 60 to 80% in 6 to 8 hours, and 80 to 100% in 8 to 10 hours.

**18.** A composition as claimed in claim 16 or claim 17, wherein the beads comprise increasing doses of drug.

**19.** A composition as claimed in claim 18, wherein the beads comprise increasing doses of drug selected from 1 mg, 2 mg, 5 mg and 10 mg, increasing to 40 mg.

**20.** A composition as claimed in any one of claims 16 to 19, wherein the beads are coated with a release rate controlling polymer.

**21.** A composition as claimed in any one of claims 16 to 20, wherein the beads comprise a central core comprising the drug and pharmaceutically acceptable composition forming ingredients.

**22.** A composition as claimed in claim 21, wherein the pharmaceutically acceptable composition forming ingredients comprise a lubricant, antioxidant and a buffer.

**23.** A composition as claimed in any one of claims 1 to 8, wherein said dosage form comprises a concentration gradient of said drug coated on a polymer substrate from a first dose of I mg to a second dose of 100 mg.

**24.** A composition as claimed in claim 23, wherein said polymer substrate comprises an erodible polymer.

**25.** A composition as claimed in claim 24, wherein the erodible polymer is a polypeptide, collagen, gelatin or polyvinyl alcohol.

**26.** A composition as claimed in claim 23, wherein said polymer substrate comprises a non-erodible polymer.

**27.** A composition as claimed in any one of claims 23 to 26, wherein the coated polymer substrate is rolled about itself such that the first dose is exposed at an outer end of the substrate and the second dose is disposed at the centre of the dosage form.

**28.** A composition as claimed in any one of claims 1 to 8, wherein said dosage form comprises a multiplicity of layers, each layer comprising an increasing dose of said drug.

**29.** A composition as claimed in claim 28, wherein the multiplicity of layers comprises 2 to 6 layers in contacting lamination.

**30.** A composition as claimed in claim 29, wherein the layers are positioned consecutively to define a laminate comprising a first exposed layer, a sixth layer in contact with the fifth layer and having its exposed surface coated with a drug impermeable polymer.

**31.** A composition as claimed in claim 30, wherein said first layer comprises 1 mg to 5 mg of said drug and each successive layer comprises an additional 1 to 5 mg of said drug.

**32.** A composition as claimed in any one of claims 28 to 30, wherein a first layer of said dosage form comprises 100 ng of said drug and successive layers comprise an increase of from 5 to 50 % by weight of said drug.

**33.** A composition as claimed in any one of claims 28 to 32, wherein each layer comprises a biodegradable polymer.

**34.** A composition as claimed in claim 33, wherein the biodegradable polymer is selected from the group consisting of poly(amides), poly(amino acids), poly(esters), poly(lactic acid), poly(glycolic acid), poly(orthoesters), poly(anhydrides), poly(dehydropyrans) and poly(dioxinones).

**35.** A composition as claimed in any one of claims 1 to 8, wherein said dosage form comprises a polymer capable of releasing said drug by diffusion.

**36.** A composition as claimed in any one of claims 1 to 8, wherein said dosage form comprises a polymer capable of releasing said drug by flux through pores.

**37.** A composition as claimed in any one of claims 1 to 8, wherein said dosage form comprises a polymer matrix capable of releasing said drug by rupture of said matrix.

**38.** Use of a central nervous system stimulant in the manufacture of a dosage form as defined in any one of claims 1 to 37 for use in the treatment of an Attention-Deficit Disorder.

**39.** Use as claimed in claim 38, wherein said dosage form provides a sustained and increasing dose of 100 ng to 500 mg of said drug over a period of 16 hours.

**40.** Use as claimed in claim 38, wherein said dosage form provides a sustained and increasing dose of 100 ng to 375 mg of said drug over a period of 16 hours.

**41.** Use of a central nervous system stimulant in the manufacture of a dosage form as defined in any one of claims 1 to 37 for use in a method of compensating for acquired tolerance to said drug.

**42.** Use of a central nervous system stimulant in the manufacture of a dosage form as defined in any one of claims 1 to 37 for regulation of tolerance to said drug.

**43.** A pharmaceutical composition in a dosage form, comprising a dose of drug in a concentration gradient from a lower to a higher dose that is released in a lower to a higher dose by the dosage form.

FIG. 1

FIG. 2

EP 1 690 531 A2

FIG. 3

FIG. 4

EP 1 690 531 A2

FIG. 5

FIG. 6

FIG. 7

FIG. 8

FIG. 9

FIG. 10

EP 1 690 531 A2

FIG. 11

EP 1 690 531 A2

EP 1 690 531 A2

FIG. 12

FIG. 13

FIG. 14

EP 1 690 531 A2

FIG. 15

FIG. 16